# EUROPEAN PATENT APPLICATION

(11) **EP 1 932 478 A1**
(43) Date of publication of application: **18.06.2008**
(21) Application number: 07023822.5
(22) Date of filing: 10.12.2007
(51) Int. Cl.: A61B 8/08

(54) **Ultrasound diagnostic system and method of displaying multiple growth trend charts**

(30) Priority: 12.12.2006 KR 20060126395
(71) Applicant: MEDISON CO., LTD., Kangwon-do 250-870 (KR)
(72) Inventor: Choi, Yae Chan, Gangnam-gu Seoul 135-280 (KR)
(74) Representative: Schmid, Wolfgang

(57) **Abstract**

An ultrasound diagnostic system, includes: an ultrasound image forming unit configured to form an ultrasound image of a target object based on ultrasound echo signals reflected from the target object; a measuring unit configured to repeatedly measure the anatomical components of the target object on the ultrasound image to provide measurement data; a user input unit configured to receive information of respective measuring time points of said measurements; a storing unit configured to cumulatively store the measurement data in association with the respective measuring time points; a chart forming unit configured to form a plurality of growth trend charts showing the cumulatively stored measurement data over the measuring time points; and a display unit configured to display the plurality of growth trend charts at the same time.

## Description

The present application claims priority from Korean Patent Application No. 10-2006-0126395 filed on December 12, 2006, the entire subject matter of which is incorporated herein by reference.

### BACKGROUND

### 1. Field

The present invention generally relates to ultrasound diagnostic fields, and more particularly to an ultrasound diagnostic system and method of simultaneously displaying multiple growth trend charts showing various anatomical components of a fetus over a gestational age.

### 2. Background

An ultrasound diagnostic system has become an important and popular diagnostic tool since it has a wide range of applications. Specifically, due to its non-invasive and non-destructive nature, the ultrasound diagnostic system has been extensively used in the medical profession. Modern high-performance ultrasound diagnostic systems and techniques are commonly used to produce two or three-dimensional images of internal features of an object (e.g., human organs).

The ultrasound diagnostic system generally uses a probe containing a wide bandwidth transducer to transmit and receive ultrasound signals. The ultrasound diagnostic system forms images of human internal tissues by electrically exciting an acoustic transducer element or an array of acoustic transducer elements to generate ultrasound signals that travel into the body. The ultrasound signals produce ultrasound echo signals since they are reflected from body tissues, which appear as discontinuities to the propagating ultrasound signals. Various ultrasound echo signals return to the transducer element and are converted into electrical signals, which are amplified and processed to produce ultrasound data for an ultrasound image of the tissues.

Recently, the ultrasound diagnostic system provides growth trend charts as a function of a gestational age. The trend charts depict measurement data of various anatomical components of a fetus such as an estimated fetal weight (EFW), a biparietal diameter (BPD), a femur length (FL), an abdomen circumference (AC), etc. of a fetus as the function of a gestational age over the course of a gestation. In order to provide the growth trend charts, the anatomical components of the fetus are repeatedly measured on an ultrasound image over the gestational age (weeks or months). The measurement data are cumulatively stored in the ultrasound diagnostic system. The stored data may be used to form a growth trend chart showing a change of each anatomical component of the target object over the gestational age. Thus, the user can intuitively perceive the trend of each anatomical component of the fetus.

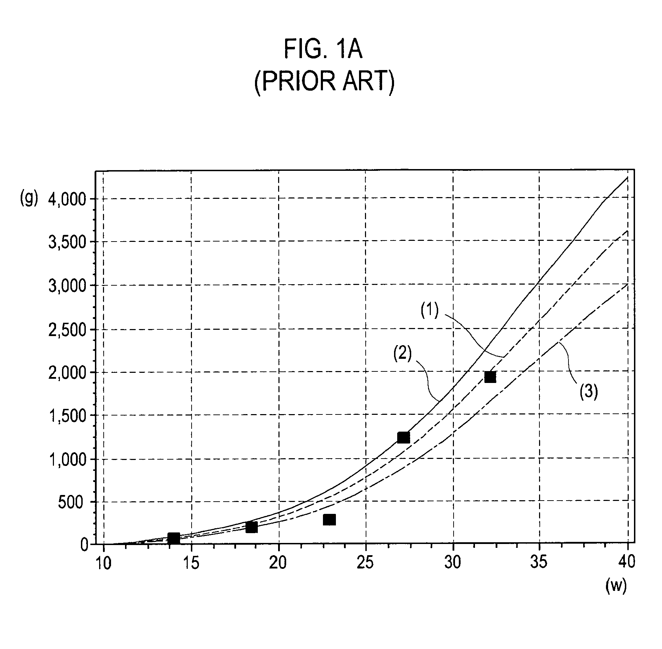

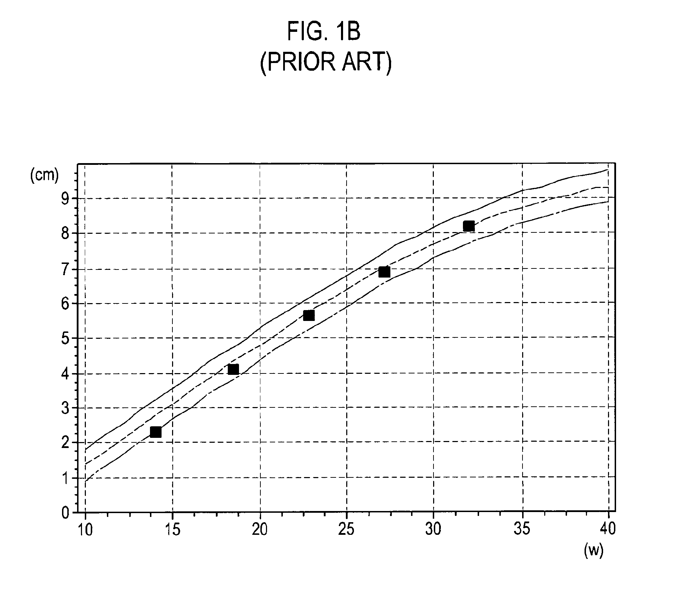

FIGS. 1A and 1B show examples, which illustrate the growth trend charts of an estimated fetal weight (EFW) and a biparietal diameter (BPD) over the gestational age (weeks), respectively, according to the prior art. The growth trend chart shows accumulated data, which are repeatedly acquired by measuring the anatomical components of the fetus. In the growth trend chart, a curve (1) represents the mean of measurement data of the anatomical component over the gestational age and curves (2) and (3) represent a variation to be considered as a normal condition. The plotted squares represent the measurement data.

As shown in FIGS. 1A and 1B, only one growth trend chart for a specific anatomical component of the fetus is provided on a screen in the conventional ultrasound diagnostic system. Thus, it is difficult to observe a trend of the entire anatomical components of the fetus over the course of a gestation at one time.

### BRIEF DESCRIPTION OF THE DRAWINGS

Arrangements and embodiments may be described in detail with reference to the following drawings in which like reference numerals refer to like elements and wherein:

FIGS. 1A and 1B show examples showing the growth trend charts of an estimated fetal weight (EFW) and a biparietal diameter (BPD) over the gestational age (weeks), respectively, according to the prior art;

FIG. 2 is a block diagram showing an ultrasound diagnostic system in accordance with the present invention;

FIG. 3 is a diagram showing an example of a window for selecting anatomical components desired to form growth trend charts in accordance with the present invention;

FIG. 4 is a diagram showing an example of displaying a plurality of growth trend charts at the same time in accordance with the present invention; and

FIG. 5 is a flowchart showing a method of simultaneously displaying a plurality of growth trend charts at the same time in accordance with the present invention.

### DETAILED DESCRIPTION

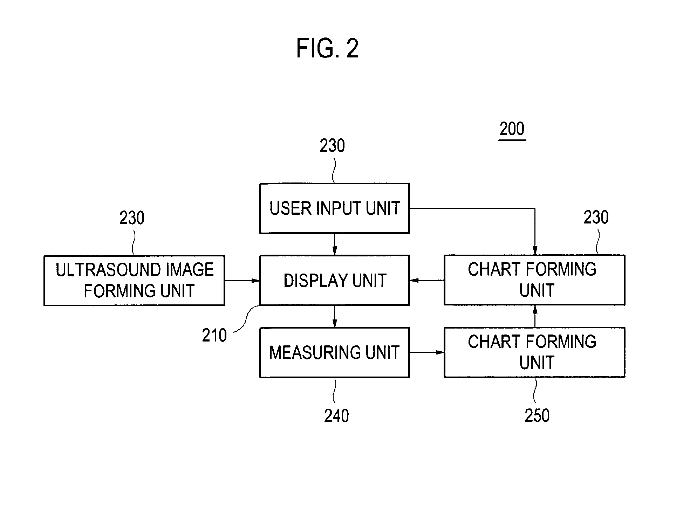

FIG. 2 is a block diagram showing an ultrasound diagnostic system, which is constructed in accordance with the present invention. The ultrasound diagnostic system 200 includes an ultrasound image forming unit 210, a display unit 220, a user input unit 230, a measuring unit 240, a storing unit 250 and a chart forming unit 260.

The ultrasound image forming unit 210 transmits ultrasound signals to the target object and then receives ultrasound echo signals reflected from the target object. The ultrasound image forming unit 210 forms an ultrasound image of the target object based on the received ultrasound echo signals. The ultrasound image is displayed on a screen of the display unit 220.

The user input unit 230 may include a track ball mounted on a control panel, a mouse, a keyboard, etc. in the ultrasound diagnostic system 200. The user input unit 230 receives measurement information to measure an anatomical component desired to measure on the ultrasound image displayed in the display unit 220. The user input unit 230 further receives information upon the gestational age (weeks, months, etc.) of the target object. If the measurement information to measure the anatomical component is inputted through the user input unit 230, a marker for measuring the desired anatomical component is activated on the ultrasound image displayed in the display unit 220. A shape or size of the marker may be adjusted by using the track ball, the mouse, the keyboard, etc. on the displayed ultrasound image. The marker is set on the anatomical component desired to measure on the ultrasound image. If the marker is completely set, then the measuring unit 240 measures the anatomical component based on the set marker.

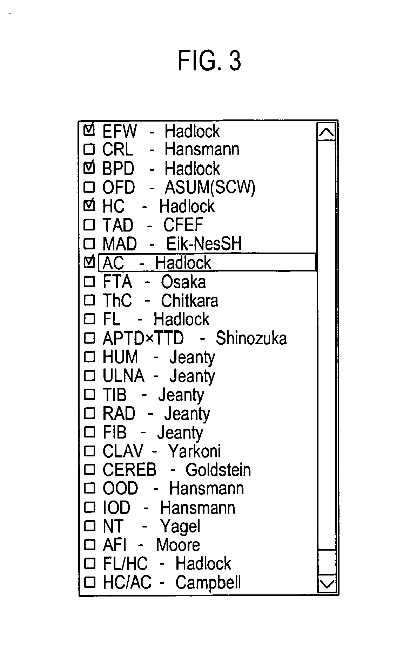

The storing unit 250 stores measurement data acquired by measuring the anatomical component in the measuring unit 240. The measurement data may be cumulatively stored in the storing unit 250 in association with the gestational age (days, weeks, months, etc). If a selection instruction to select an anatomical component for displaying a growth trend chart thereof is inputted through the user input unit 230, then the chart forming unit 250 extracts data corresponding to the selected anatomical component from the storing unit 140. The selection of the anatomical component may be achieved by using a menu window such as a pop-up window provided on a screen of the display unit 220 as shown in FIG. 3. Check buttons or radio buttons may be provided on the menu window so as to select each anatomical component. When an arbitrary anatomical component is selected, the selection may be indicated on the check button or the radio button so as to recognize which anatomical component is selected. Also, the menu window displaying items of the anatomical components for the growth trend charts may be provided on a touch screen mounted on the control panel of the ultrasound diagnostic system. A plurality of anatomical components may be selected by using the track ball in the control panel, the mouse, the keyboard, etc.

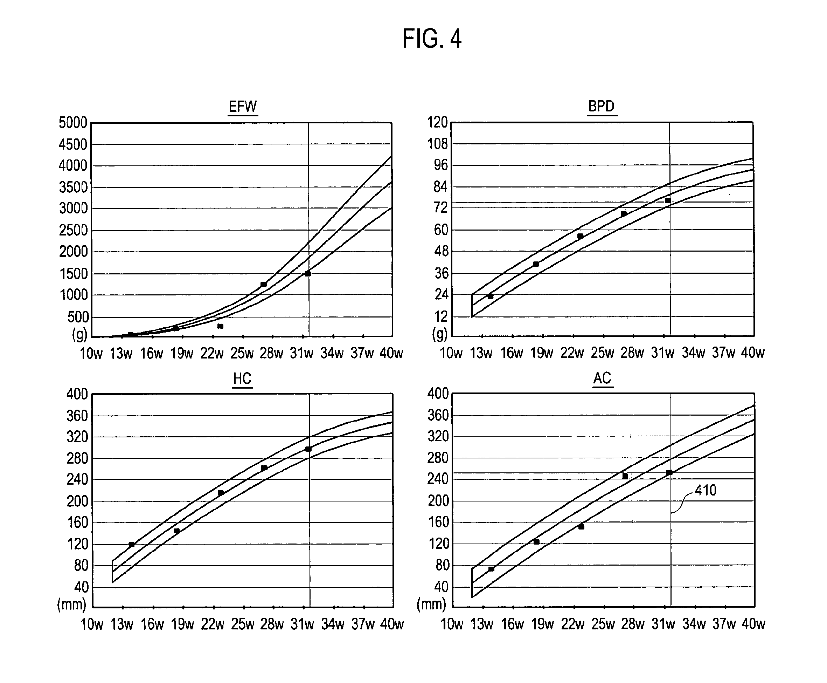

The chart forming unit 260 forms growth trend charts of the anatomical components, which are selected in response to the selection instruction, in predetermined forms based on the extracted data. The display unit 220 may display the growth trend charts for the respective selected anatomical components, which are formed at the chart forming unit 260, on a screen at the same time as shown in FIG. 4. As shown in FIG. 4, there are the growth trends charts showing measurement data of estimated fetal weight (EFW), biparietal diameter (BPD), femur length (FL) and abdomen circumference (AC), which are displayed on a screen at the same time, respectively.

In accordance with the present invention, if an arbitrary gestational age (days, weeks or months) is selected on an arbitrary growth trend chart among the plurality of growth trends charts, then a reference line 410, which shows the selected gestational age and the measurement data, is generated and displayed on the corresponding growth trend chart. The selection of the gestational age may be achieved by using one of the track ball, the mouse and the keyboard. The reference line 410 may be displayed to be perpendicular to a horizontal axis representing the gestational age. The information corresponding to the selected gestational age can be easily observed through the reference line 410. This is so that the user rapidly and accurately determines whether or not the corresponding anatomical component is in a normal condition. In such a case, if the user selects a specific gestational age on an arbitrary growth trend chart, then the reference lines are simultaneously set not only on the selected growth trend chart but also on other growth trend charts displayed on the screen of the display unit 220. Thus, the user can easily observe measurement data of all anatomical components of the target object without any manipulations.

Also, if a moving instruction for moving one of the reference lines to the other gestational age is inputted, then all reference lines displayed on the respective growth trend charts are identically moved such that the reference lines indicate the same gestational age in the respective growth trend charts. Thus, since a plurality of growth trend charts are displayed at the same time and the reference lines are identically moved to indicate the same gestational age, the user can intuitively perceive which anatomical component of the fetus is in a normal condition or an abnormal condition.

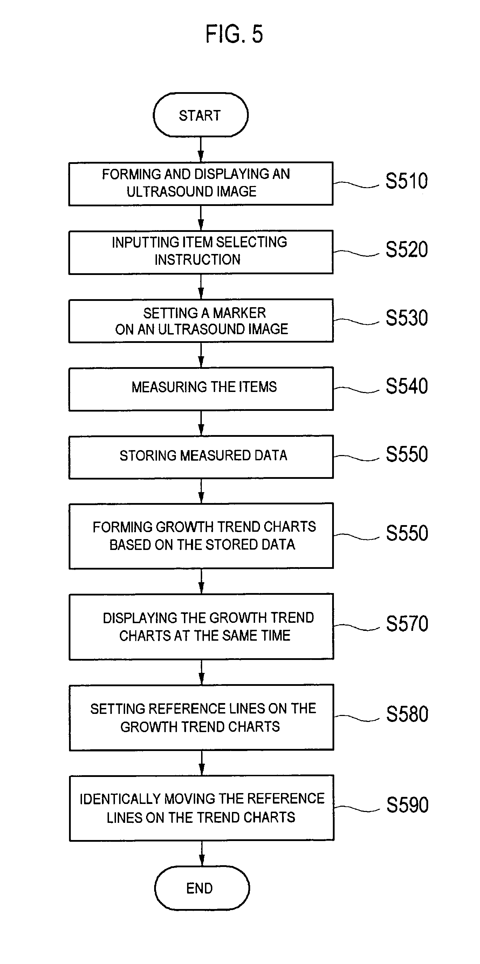

Hereinafter, a method of displaying growth trend charts of the target object will be described with reference to FIG. 5 in accordance with one embodiment of the present invention. FIG. 5 is a flowchart showing the method of displaying the growth trend charts of the target object in accordance with one embodiment of the present invention.

Referring to FIG. 5, an ultrasound image of the target is formed based on ultrasound echo signals reflected from the target object and the ultrasound image is displayed at step S510. After displaying the ultrasound image of the target object, a selection instruction for selecting an anatomical component desired to measure in the target object is inputted at step S520. When the target object is a fetus, the anatomical component to be measured may be an estimated fetal weight (EFW), a biparietal diameter (BPD), a femur length (FL), an abdomen circumference (AC), etc. Also, information of the target object may be inputted. The information of the target object may include a gestational age represented as days, weeks or months.

Subsequently, a marker necessary for measuring the anatomical component of the target object is set in response to the selection instruction on the ultrasound image at step S530. The marker may be adjusted by using one of track ball, mouse and keyboard to match the marker with the corresponding part on the ultrasound image at step S540. The measurement of each anatomical component of the target object may be repeatedly carried out. The measurement data are cumulatively stored with the information of the target object at step S550.

Thereafter, if a selection instruction for selecting a plurality of anatomical components to form growth trend charts thereof is inputted, then the growth trend charts of the selected anatomical components are formed based on the stored data at step S560. The formed growth trend charts are displayed on the screen of the display unit 220 at the same time at step S570. Subsequently, if an instruction for selecting a specific gestational age (days, weeks or months) at an arbitrary growth trend chart is inputted, reference lines are set at the selected gestational age on all growth trend charts, which are currently displayed, at step S580. If a moving instruction for selecting and moving the reference line at an arbitrary growth trend chart is inputted, then not only the selected reference line but also the other reference lines are identically moved such that all the reference lines indicate the same gestational age at step S590.

As mentioned above, since the ultrasound diagnostic system can display a plurality of growth trend charts showing the measurement data of the various anatomical components of the fetus in accordance with the present invention, a general change of the fetus can be easily observed. Also, when the user selects a specific gestational age (weeks or months), a vertical reference line is automatically set with respect to the specific gestational age in each growth trend chart at the same time. As such, this makes it easy to determine whether or not a specific anatomical component is normal.

In accordance with one embodiment of the present invention, there is provided an ultrasound diagnostic system, including: an ultrasound image forming unit configured to form an ultrasound image of a target object based on ultrasound echo signals reflected from the target object; a measuring unit configured to repeatedly measure the anatomical components of the target object on the ultrasound image to provide measurement data; a user input unit configured to receive information of respective measuring time points of said measurements; a storing unit configured to cumulatively store the measurement data in association with the respective measuring time points; a chart forming unit configured to form a plurality of growth trend charts showing the cumulatively stored measurement data over the measuring time points; and a display unit configured to display the plurality of growth trend charts at the same time.

In accordance with another embodiment of the present invention, there is a provided a method of simultaneously displaying a plurality of growth trend charts in an ultrasound diagnostic system, comprising: forming an ultrasound image of a target object based on ultrasound echo signals reflected from the target object; repeatedly measuring the anatomical components of the target object on the ultrasound image to provide measurement data; receiving information of respective measuring time points of said measurements; cumulatively storing the measurement data in association with the respective measuring time points; forming a plurality of growth trend charts showing the cumulatively stored measurement data over the measuring time points; and displaying the plurality of growth trend charts at the same time.

Any reference in this specification to "one embodiment," "an embodiment," "example embodiment," etc., means that a particular feature, structure or characteristic described in connection with the embodiment is included in at least one embodiment of the present invention. The appearances of such phrases in various places in the specification are not necessarily all referring to the same embodiment. Further, when a particular feature, structure or characteristic is described in connection with any embodiment, it is submitted that it is within the purview of one skilled in the art to effect such feature, structure or characteristic in connection with other ones of the embodiments.

Although embodiments have been described with reference to a number of illustrative embodiments thereof, it should be understood that numerous other modifications and embodiments can be devised by those skilled in the art that will fall within the spirit and scope of the principles of this disclosure. More particularly, numerous variations and modifications are possible in the component parts and/or arrangements of the subject combination arrangement within the scope of the disclosure, the drawings and the appended claims. In addition to variations and modifications in the component parts and/or arrangements, alternative uses will also be apparent to those skilled in the art.

## Claims

1. An ultrasound diagnostic system, comprising:
an ultrasound image forming unit configured to form an ultrasound image of a target object based on ultrasound echo signals reflected from the target object;
a measuring unit configured to repeatedly measure the anatomical components of the target object on the ultrasound image to provide measurement data;
a user input unit configured to receive information of respective measuring time points of said measurements;
a storing unit configured to cumulatively store the measurement data in association with the respective measuring time points;
a chart forming unit configured to form a plurality of growth trend charts showing the cumulatively stored measurement data over the measuring time points; and
a display unit configured to display the plurality of growth trend charts at the same time.

2. The ultrasound diagnostic system of Claim 1, wherein the user input unit further receives a first selection instruction for selecting specific anatomical components, the chart forming unit forms growth trend charts of the selected anatomical components, and the display unit displays the growth trend charts of the selected anatomical components at the same time.

3. The ultrasound diagnostic system of Claim 2, wherein the user input unit further receives a second selection instruction for selecting a specific measuring time point on the growth trend charts, and the display unit displays reference lines indicating the selected measuring time point and the measurement data corresponding to the selected measuring time point on the entire growth trend charts.

4. The ultrasound diagnostic system of Claim 3, wherein the user input unit further receives a moving instruction for moving the reference lines on the growth trend charts, and the reference lines of the respective growth trend charts are identically moved on the growth trend charts in response to the moving instruction such that the moved reference lines indicate the same measuring point.

5. A method of simultaneously displaying a plurality of growth trend charts in an ultrasound diagnostic system, comprising:
forming an ultrasound image of a target object based on ultrasound echo signals reflected from the target object;
repeatedly measuring the anatomical components of the target object on the ultrasound image to provide measurement data;
receiving information of respective measuring time points of said measurements;
cumulatively storing the measurement data in association with the respective measuring time points;
forming a plurality of growth trend charts showing the cumulatively stored measurement data over the measuring time points; and
displaying the plurality of growth trend charts at the same time.

6. The method of Claim 5, further comprising:
receiving a first selection instruction for selecting specific anatomical components;
forming growth trend charts of the selected anatomical components;
displaying the growth trend charts of the selected anatomical components at the same time.

7. The method of Claim 6, further comprising:
receiving a second selection instruction for selecting a specific measuring time point on the trend charts; and
displaying reference lines indicating the selected measuring time point and the measurement data corresponding to the selected measuring time point on the entire growth trend charts.

8. The method of Claim 7, further comprising receiving a moving instruction for moving the reference lines on the trend charts, wherein the reference lines of the respective trend charts are identically moved on the growth trend charts in response to the moving instructions such that the moved reference lines indicate the same measuring time point.
